# EUROPEAN PATENT APPLICATION

(11) **EP 1 614 698 A1**
(43) Date of publication of application: **11.01.2006**
(21) Application number: 05291207.8
(22) Date of filing: 06.06.2005
(51) Int. Cl.: C08F 4/34, C08F 14/06, C07C 409/00

(54) **Initiator compositions**

(30) Priority: 09.07.2004 US 888053
(71) Applicant: Arkema Inc., Philadelphia, PA 19103-3222 (US)
(72) Inventor: Stainbrook, Barbara L., Collegeville Pennsylvania (US); Mendolia, Michael, Philadelphia Pennsylvania (US); Myers, Terry N., Phoenixville Pennsylvania (US); Callais, Peter A., Collegeville Pennsylvania (US)
(74) Representative: Lhoste, Catherine

(57) **Abstract**

Peroxide/diluent initiator compositions are provided which reduce the formation of VOCs during the manufacture of ethylenically unsaturated monomer, oligomer and polymer resins.

## Description

### FIELD OF THE INVENTION

This invention relates to initiator compositions useful for the polymerization of ethylenically unsaturated monomers such as vinyl chloride, modifying polyolefins and elastomers and curing of unsaturated polyester resins, particularly to the reduction of VOC's in such processes using organic peroxide based initiator compositions.

### BACKGROUND OF THE INVENTION

Odorless mineral spirits (OMS) is a standard diluent used for incorporation into a peroxide initiator during manufacture or prior to its use. Diluents are useful for better processing, safety, and the like. A problem with the use of OMS is that it does not polymerize with the monomers, oligomers and polymers and therefore is volatilized during processing, causing VOC emissions. In addition, certain diluents may be altered during the peroxide manufacturing process. Such alteration can negate the positive safety, processing etc. aspects of the diluent. It would accordingly be useful to find a diluent which does not volatilize during processing of ethylenically unsaturated monomers, oligomers and polymers and which can be incorporated into the peroxide composition at any point, prior to manufacture of the peroxide, during its manufacture, or subsequent to its manufacture.

Diluents have been previously proposed, as in U.S. Patents 4,131,728 and 4,178,263. However, the previously proposed diluents may exhibit limitations and not all of these diluents are suitable for the manufacture of peroxides. For example, solid diluents do not allow aqueous separations and other of the monomeric diluents are altered by the preparative process used to make the peroxide.

### DETAILED DESCRIPTION

Initiator compositions for ethylenically unsaturated monomer, oligomer and polymer resin manufacture are provided, which compositions comprise (a) a peroxide selected from the group consisting of peroxyesters, monoperoxycarbonates, dialkyl peroxides, diacyl peroxides and peroxyketals, and (b) a diluent of the formula R₁OC(O)CH=CHC(O)OR₂ where R₁ and R₂ are the same or different and are selected from the group consisting of alkyl of 1-20 carbons, cycloalkyl of 5-10 carbons and aralkyl of 7-11 carbons, as well as use of these compositions as an initiator for the polymerization of ethylenically unsaturated monomers, and modification of oligomers and polymers. When present during the manufacture of the peroxide, the R groups each contain at least 4 carbons.

Representative examples of peroxides in accordance with the present invention include: peroxyesters such as t-amyl peroxypivalate, t-butyl peroxyneodecanoate, t-butyl peroxyneoheptanoate, t-butyl peroxy-2-ethylhexanoate, 3-hydroxy-1,1-dimethylbutyl peroxy-2-ethylhexanoate, 3-hydroxy-1,1-dimethylbutyl peroxyneoheptanoate, t-amyl peroxy-2-ethylhexanoate, t-butyl peroxybenzoate, t-amyl peroxybenzoate, 2,5-dimethyl-2,5-hexanediyl bis(peroxy-2-ethylhexanoate), t-amyl peroxyacetate, t-butyl peroxy-2-methylbenzoate, t-butyl peroxymaleic acid, 1,1,3,3-tetramethylbutyl peroxyneodecanoate, and t-butyl peroxyisobutyrate; monoperoxycarbonates such as: OO-(t-butyl) O-(2-ethylhexyl) monoperoxycarbonate, OO-(t-amyl) 0-(2-ethylhexyl) monoperoxycarbonate, OO-(t-butyl) O-isopropyl monoperoxycarbonate, OO-(t-amyl) O-isopropyl monoperoxycarbonate, Polymonoperoxycarbonates as described in US 5,760,149 (incorporated herein by reference), and OO-(t-butyl) O-stearyl monoperoxycarbonate; dialkyl peroxides such as: di(t-amyl) peroxide, di(t-butyl) peroxide, 2,5-dimethyl-2,5-di(t-butylperoxy)hexane, 1,3(4)-di(1-(t-butylperoxy)-1-methylethyl)benzene mixture (or pure isomers), dicumyl peroxide, 2,5-dimethyl-2,5-di(t-butylperoxy)-3-hexyne, and di(1,1,3,3-tetramethylbutyl) peroxide; diacyl peroxides such as: diisobutyryl peroxide, diisononanoyl peroxide, didodecanoyl peroxide (dilauroyl peroxide), didecanoyl peroxide, succinic acid peroxide (di(3-carboxypropanoyl) peroxide), and di(2-ethylhexanoyl) peroxide; and perketals such as: 1,1-di(t-butylperoxy)cyclohexane, 1,1 -di(t-amylperoxy)cyclohexane , 1,1 -di(t-butylperoxy)-3,5,5-trimethylcyclohexane, ethyl 3,3-di(t-butylperoxy)butyrate, ethyl 3,3-di(t- amylperoxy)butyrate, n-butyl 4,4-di(t-butylperoxy)valerate, n-butyl 4,4- di(t-amylperoxy)valerate, 2,2-di(4,4-di(t-butylperoxy)cyclohexyl)proapane, 2,2-di(t-amylperoxy)propane, and 2,2-di(t-butylperoxy)butane.

The peroxide initiator is combined with a diluent in the initiator composition of the present invention. Preferred diluents include di(n-butyl) fumarate, distearyl fumarate, diethyl fumarate, dimethyl maleate, dicyclohexyl fumarate, dibenzyl maleate, dieicosyl fumarate, didodecyl fumarate, n-butyl 3-(ethoxycarbonyl)propenoate, benzyl 3-(hexoxycarbonyl)propenoate, and mixtures thereof, including blends of symmetrical and unsymmetrical fumarates and maleates such as di(n-butyl)maleate/dimethyl maleate/ n-butyl 3-(methoxycarbonyl)propenoate.

Preferred examples of diluted peroxide formulations in accordance with the present invention include, for example: t-amyl peroxyacetate, 55% in dibutyl fumarate; OO-(t-butyl) O-isopropyl monoperoxycarbonate, 75% in diethyl fumarate; 1,1-di(t-butylperoxy)cyclohexane, 75% in dibutyl maleate; diisononanoyl peroxide, 60% in dibutyl maleate; t-butyl peroxyneodecanoate, 75% in diethyl maleate; Dicumyl peroxide in mixed fumarates comprising di(n-butyl) fumarate, di(isopropyl) fumarate, and n-butyl 3-(isopropoxycarbonyl)propenoate.

It has now been found that the foregoing compositions do not contribute to VOCs in products prepared using them, for example PVC manufacture. In the manufacturing process, the diluent is actually polymerized with the ethylenically unsaturated monomer to make the desired polymer, or otherwise incorporated into the oligomer and polymer during the modification process. Further, those diluents in which the R groups have at least 4 carbons are not altered when present during preparation/manufacturing of the peroxides.

The aforementioned peroxides and their method of preparation are well known in the art. The dilutions of the peroxides can be accomplished at any convenient stage in the process, before, during or after their manufacture. When the diluent is required to survive processing steps at high pH, R groups of at least 4 carbons are used to avoid hydrolysis of the ester groups which would lead to removal of the diluent during aqueous separation steps in the preparation of the peroxide. The importance of the size of the R groups is illustrated in Example 1.

### EXAMPLES

Example 1: Use of Diluent in Processing of t-Butyl Peroxypivalate:

Synthesis of t-Butyl Peroxypivalate was undertaken with OMS (a commercial diluent), diethyl maleate and dibutyl maleate as a diluent. The diluent was present during the preparation of the t-Butyl Peroxypivalate. The peroxide assay, after the synthesis was complete, was about 78% for OMS. In the synthesis of t-Butyl Peroxypivalate using diethyl maleate (R groups have 2 carbons) and dibutyl maleate (R groups have 4 carbons) as diluent, the peroxide assay, after the synthesis was about 78% for the dibutyl maleate, while the peroxide assay was about 91 % for diethyl maleate. The results show that the diethyl maleate diluent was altered and/or washed away, providing an unacceptably high peroxide assay result. The peroxide assay for dibutyl maleate was the same as the currently used diluent OMS.

Example 2:

Use of the diluent dibutyl maleate was compared to OMS in the processing of three additional peroxides: a-cumyl peroxyneodecanoate; 1,1-dimethyl-3-hydroxybutyl peroxyneodecanoate; and a-cumyl peroxyneoheptanoate. With the diluent present during the peroxide synthesis process, the peroxide assay results with dibutyl maleate mirrored the assay results with OMS. For 1,1-dimethyl-3-hydroxybutyl peroxyneodecanoate synthesis, the peroxide assay was about 76% for both OMS and dibutyl maleate. For 1,1-dimethyl-3-hydroxybutyl peroxyneodecanoate synthesis, the peroxide assay was about 55% for both OMS and dibutyl maleate. For a -cumyl peroxyneoheptanoate synthesis, the peroxide assay was about 78% for both OMS and dibutyl maleate. The results show that dibutyl maleate is not adversely affected by the peroxide synthesis process and provides dilution efficacy comparable to a current commercial material OMS.

Example 3:

OMS or dibutyl maleate was added as a diluent after preparation of the peroxide, t-amyl peroxyneodecanoate. A peroxide assay was performed. Again, the assay results with dibutyl maleate mirrored the assay results with OMS, with both providing a peroxide assay of about 82%.

Example 4:

The polymerization of Vinyl Chloride was performed using peroxides diluted with a traditional diluent, Odorless Mineral Spirits (OMS), and a diluent of the present invention, dibutyl maleate. The jacketed polymerization vessel employed in the vinyl chloride suspension polymerizations was equipped with a pressure gauge, mechanical stirrer, cooling coils and thermocouples for various temperature measurements. Water and the polyvinyl alcohols were added to the vessel. The vessel was heated to 58°C and the required amount of vinyl chloride monomer and 0.001 % by weight of initiator samples was added to the vessel. The time to pressure drop was measured and taken as the end of the polymerization reaction. A sample of the water and the polymer were analyzed for residual diluent. As shown in Table 1, a diluent in accordance with the present invention provided a lower level of residual solvent than the current commercial diluent OMS.

**TABLE 1**

| Initiator | Extractables | |
|---|---|---|
| | OMS | Dibutyl Maleate |
| Luperox 188 75 % in OMS | 190 ppm | -- |
| Luperox 223 75 % in OMS | | |
| Luperox 188 75 % in dibutyl maleate | -- | <5 ppm |
| Luperox 223 75 % in dibutyl maleate | | |

Luperox 188 is α-cumyl peroxyneodecanoate and Luperox 223 is di(2-ethylhexyl) peroxydicarbonate, both available from ATOFINA Chemicals of Philadelphia, PA.

While the present invention has been described with respect to particular embodiments thereof, it is apparent that numerous other forms and modifications of this invention will be obvious to those skilled in the art. The appended claims and this invention generally should be construed to cover all such obvious forms and modifications which are within the true spirit and scope of the present invention.

## Claims

1. An initiator composition comprising (a) a peroxide selected from the group consisting of peroxyesters, monoperoxycarbonates, dialkyl peroxides, diacyl peroxides and peroxyketals, and (b) a diluent of the formula R₁ OC(O)CH=CHC(O)OR₂ where R₁ and R₂ are the same or different and are selected from the group consisting of alkyl of 1-20 carbons, cycloalkyl of 5-10 carbons and aralkyl of 7-11 carbons.

2. A process for preparing an initiator composition of Claim 1 wherein the diluent is present during manufacture of the peroxide and wherein R₁ and R₂ each contain at least 4 carbons.

3. Use of the composition of Claim 1 as an initiator for the polymerization of ethylenically unsaturated monomers or mixtures.

4. Use of the composition of Claim 1 as an initiator for the polymerization of vinyl chloride monomer, and others.

5. An organic peroxide composition comprising (a) a peroxide selected from the group consisting of peroxyesters, monoperoxycarbonates, dialkyl peroxides, diacyl peroxides and peroxyketals, and (b) a diluent of the formula R₁OC(O)CH=CHC(O)OR₂ where R₁ and R₂ are the same or different and are selected from the group consisting of alkyl of 4-20 carbons, cycloalkyl of 5-10 carbons and aralkyl of 7-11 carbons.
